# EUROPEAN PATENT APPLICATION

(11) **EP 4 537 814 A1**
(43) Date of publication of application: **16.04.2025**
(21) Application number: 23819256.1
(22) Date of filing: 09.06.2023
(51) Int. Cl.: A61K 9/08, A61K 47/06, A61K 31/519, A61P 35/00

(54) **PEMETREXED DISODIUM LIQUID COMPOSITION, PREPARATION METHOD THEREFOR AND USE THEREOF**

(30) Priority: 09.06.2022 CN 202210645147
(71) Applicant: Shanghai Aurora Biotechnology Co., Ltd., Shanghai 201210 (CN)
(72) Inventor: FU, Jun, Shanghai 201210 (CN); GUO, Zhen, Shanghai 201210 (CN); JIN, Haigang, Shanghai 201210 (CN); CHEN, Zhixiang, Shanghai 201210 (CN); HUANG, Anping, Shanghai 201210 (CN); LIU, Shuang, Shanghai 201210 (CN); WANG, Tingting, Shanghai 201210 (CN); YING, Shuhuan, Shanghai 201210 (CN)
(74) Representative: Gille Hrabal Partnerschaftsgesellschaft mbB Patentanwälte
(86) International application number: PCT/CN2023/099336
(87) International publication number: WO 2023/237093

(57) **Abstract**

Disclosed are a pemetrexed disodium liquid composition, a preparation method therefor and use thereof. The pemetrexed disodium liquid composition provided in the present invention comprises: an active pharmaceutical ingredient and a stabilizer. The active pharmaceutical ingredient is one or more of pemetrexed disodium, and a pharmaceutically acceptable complex, a salt, a solvate and a hydrate of pemetrexed disodium. The stabilizer is one or more of an organic solvent, a pH regulator and an antioxidant. The pemetrexed disodium liquid composition of the present invention has the advantages of simple preparation process, good physical and chemical stability, convenient clinical use, no need of redissolution, low microbial pollution risk in the preparation process, suitability for industrial production and the like.

## Description

The present application claims the priority to Patent Application No. 202210645147.0, entitled "PEMETREXED DISODIUM LIQUID COMPOSITION, PREPARATION METHOD THEREFOR AND USE THEREOF" filed with the China National Intellectual Property Administration on June 9, 2022, which is incorporated herein by reference in its entirety.

### TECHNICAL FIELD

The present disclosure relates to a pemetrexed disodium liquid composition, a preparation method therefor and use thereof.

### BACKGROUND

Pemetrexed is a multi-target antimetabolite anti-tumor drug and a folic acid antagonist, and can inhibit folic acid-dependent enzymes such as thymidylate synthase, dihydrofolate reductase, glycinamide ribonucleotide formyltransferase, and the like. The enzymes participate in the biosynthesis of thymidine and purine nucleoside, thereby achieving anti-tumor effect. Pemetrexed disodium was first developed by Eli Lilly and Company, was approved by the FDA in 2004, obtained the import drug permission of China in August 2005, and started to be used for clinical application in China. The drug treats unresectable malignant pleural mesothelioma in combination with cisplatin for the first line, and treats non-squamous non-small cell lung cancer as a monotherapy for the second line.

The drug, which has been marketed in multiple countries and regions worldwide, is a lyophilized powder injection. Before use, the drug is reconstituted into a concentrate with the concentration of 25 mg/mL by using 0.9% sodium chloride solution according to the specification of the drug, and then the concentrate is subjected to a second dilution for administration. The reconstitution process may lead to an error in the dose administered to a patient, causing safety concerns and the risk of microbial contamination.

Therefore, the search for a pemetrexed dosage form which is convenient to use, good in physical and chemical stability, simple in preparation process and suitable for industrial production is a technical problem which needs to be solved urgently at present.

### SUMMARY

The present disclosure provides a pemetrexed disodium liquid composition comprising an active pharmaceutical ingredient and a stabilizer, wherein the active pharmaceutical ingredient is selected from one or more of pemetrexed disodium, and a pharmaceutically acceptable complex, salt, solvate, and hydrate of pemetrexed disodium; the stabilizer is selected from one or more of an organic solvent, a pH adjuster, and an antioxidant.

According to an embodiment of the present disclosure, the content of the active pharmaceutical ingredient is 1 mg/mL to 50 mg/mL, preferably 15 mg/mL to 30 mg/mL, such as 20 mg/mL, 25.00 mg/mL, 27.57 mg/mL, 30 mg/mL, or 40 mg/mL, and the content refers to the ratio of the mass of the active pharmaceutical ingredient to the total volume of the pemetrexed disodium liquid composition. According to an embodiment of the present disclosure, the organic solvent is selected from one or more of ethanol, propylene glycol, and polyethylene glycol. For example, the polyethylene glycol is selected from polyethylene glycol 400 (PEG400) and/or polyethylene glycol 300 (PEG300).

According to some embodiments of the present disclosure, the content of the organic solvent is 70 mg/mL to 300 mg/mL, such as 90 mg/mL to 260 mg/mL, illustratively 90 mg/mL, 180 mg/mL, 260 mg/mL, or 280 mg/mL; the content refers to the ratio of the mass of the organic solvent to the total volume of the pemetrexed disodium pharmaceutical composition.

According to an embodiment of the present disclosure, the pH adjuster can be an acidic pH adjuster and/or a basic pH adjuster. For example, the acidic pH adjuster is preferably hydrochloric acid and/or citric acid, further preferably citric acid. For example, the basic pH adjuster can be one or more of sodium hydroxide, lysine, arginine, meglumine, and tromethamine, preferably tromethamine. For example, the lysine can be L-lysine and/or D-lysine. For example, the arginine can be L-arginine and/or D-arginine.

According to an embodiment of the present disclosure, the antioxidant is selected from one or more of anhydrous sodium sulfite, cysteine hydrochloride, acetyl cysteine, and methionine, further preferably cysteine hydrochloride. The content of the antioxidant is preferably 0-15 mg/mL, further preferably 0.1 mg/mL to 10 mg/mL, such as 0.3 mg/mL, 1 mg/mL, 1.63 mg/mL, or 3 mg/mL, the content refers to the ratio of the mass of the antioxidant to the total volume of the pemetrexed disodium pharmaceutical composition.

In some embodiments, in the pemetrexed disodium liquid composition, the content of the stabilizer (e.g., the basic pH adjuster) is preferably 0.5 mg/mL to 50 mg/mL, further preferably 1 mg/mL to 10 mg/mL, such as 2.4 mg/mL, and the content refers to the ratio of the mass of the stabilizer (e.g., the basic pH adjuster) to the total volume of the pemetrexed disodium liquid composition.

According to some embodiments of the present disclosure, the stabilizer comprises at least one or more of citric acid, tromethamine, cysteine hydrochloride, meglumine, and arginine; in other embodiments, the stabilizer can further comprise propylene glycol.

According to an embodiment of the present disclosure, the pH of the pemetrexed disodium liquid composition is preferably 7.5-9.5, further preferably 8.0-9.0.

According to an embodiment of the present disclosure, the pemetrexed disodium liquid composition can further comprise an osmotic pressure regulator. The osmotic pressure regulator is preferably selected from one or more of sodium chloride, mannitol, glycerol, and propylene glycol. The content of the osmotic pressure regulator is preferably 1 mg/mL to 300 mg/mL, such as 1 mg/mL to 100 mg/mL, further preferably 2 mg/mL to 30 mg/mL, such as 5.8 mg/mL, 11 mg/mL, 15 mg/mL, or 23 mg/mL, and the content refers to the ratio of the mass of the osmotic pressure regulator to the total volume of the pemetrexed disodium liquid combination. When the pemetrexed disodium liquid composition contains propylene glycol, the propylene glycol can adjust the osmotic pressure and improve the stability of the pemetrexed disodium liquid composition.

According to an embodiment of the present disclosure, the pemetrexed disodium liquid composition further comprises water, such as water for injection, for making up the volume. Illustratively, the volume was brought to 1 mL with water.

According to an embodiment of the present disclosure, the pemetrexed disodium liquid composition is selected from any one of the following compositions:
composition I comprising an active pharmaceutical ingredient, a stabilizer, and water, wherein the active pharmaceutical ingredient is pemetrexed disodium, or a pharmaceutically acceptable complex, salt, or hydrate thereof, and the stabilizer is selected from tromethamine and citric acid;
preferably, in composition I, the content of the active pharmaceutical ingredient is 25 mg/mL, and the content of tromethamine is 2.4 mg/mL;
composition II comprising an active pharmaceutical ingredient, a stabilizer, and water, wherein the active pharmaceutical ingredient is pemetrexed disodium, or a pharmaceutically acceptable complex, salt, or hydrate thereof; the stabilizer includes tromethamine and citric acid, and further includes anhydrous sodium sulfite, acetyl cysteine, sodium thiosulfate, and/or cysteine hydrochloride;
preferably, in composition II, the content of the active pharmaceutical ingredient is 20 mg/mL, 25 mg/mL, 27.57 mg/mL, 30 mg/mL, or 40 mg/mL, the content of the tromethamine is 2.4 mg/mL, the content of the anhydrous sodium sulfite is 1 mg/mL, the content of the acetyl cysteine is 1.63 mg/mL, the content of the cysteine hydrochloride is 0.3 mg/mL, and the content of the sodium thiosulfate is 1 mg/mL;
composition III comprising an active pharmaceutical ingredient, a stabilizer, and water, wherein the active pharmaceutical ingredient is pemetrexed disodium, or a pharmaceutically acceptable complex, salt, or hydrate thereof; the stabilizer includes citric acid and cysteine hydrochloride, and further includes meglumine and/or arginine;
preferably, in composition III, the content of the active pharmaceutical ingredient is 20 mg/mL, 25 mg/mL, 27.57 mg/mL, 30 mg/mL, or 40 mg/mL, the content of the cysteine hydrochloride is 0.3 mg/mL, the content of the meglumine is 2.4 mg/mL, and the content of the arginine is 2.4 mg/mL;
composition IV comprising an active pharmaceutical ingredient, a stabilizer, and water, wherein the active pharmaceutical ingredient is pemetrexed disodium, or a pharmaceutically acceptable complex, salt, or hydrate thereof; the stabilizer includes tromethamine and citric acid, and further includes propylene glycol and/or polyethylene glycol (such as polyethylene glycol 300 or polyethylene glycol 400);
preferably, in composition IV, the content of the active pharmaceutical ingredient is 20 mg/mL, 25 mg/mL, 27.57 mg/mL, 30 mg/mL, or 40 mg/mL, the content of the tromethamine is 2.4 mg/mL, the content of the propylene glycol is 70-300 mg/mL (such as 90, 180, or 260 mg/mL), and the content of the polyethylene glycol is 70-300 mg/mL (such as 90, 180, or 280 mg/mL);
composition V comprising an active pharmaceutical ingredient, a stabilizer, an osmotic pressure regulator, and water, wherein the active pharmaceutical ingredient is pemetrexed disodium, or a pharmaceutically acceptable complex, salt, or hydrate thereof; the stabilizer includes tromethamine and citric acid; the osmotic pressure regulator is selected from sodium chloride, mannitol, glycerol, and/or propylene glycol;
preferably, in composition V, the content of the active pharmaceutical ingredient is 20 mg/mL, 25 mg/mL, 27.57 mg/mL, 30 mg/mL, or 40 mg/mL, the content of the tromethamine is 2.4 mg/mL, the content of the sodium chloride is 5.8 mg/mL, the content of the mannitol is 23 mg/mL, the content of the glycerol is 15 mg/mL, and the content of the propylene glycol is 11 mg/mL;
the pH of any one of the compositions described above is 8.0-9.0.

The present disclosure further provides a preparation method for the pemetrexed disodium liquid composition described above, which can comprise a nitrogen introducing procedure; the nitrogen introducing procedure can be selected from liquid preparation nitrogen introducing and/or nitrogen filling, preferably liquid preparation nitrogen introducing and nitrogen filling. In some embodiments, the content of the dissolved oxygen introduced to the pemetrexed disodium liquid composition via nitrogen introduction is less than 5 mg/L, preferably less than 3 mg/L; the content of the dissolved oxygen refers to the ratio of the mass of oxygen to the volume of the pemetrexed disodium liquid composition. In some embodiments, headspace residual oxygen amount is less than 6%, preferably less than 4%, and the headspace residual oxygen amount refers to the volume fraction of oxygen in the mixed gas at the top interior of the sealed packaging container in direct contact with the pemetrexed disodium liquid composition.

According to an embodiment of the present disclosure, the preparation method comprises nonterminal sterilization.

The present disclosure further provides use of the pemetrexed disodium liquid composition for manufacturing an anti-tumor drug. Preferably, the anti-tumor drug can be a liquid pharmaceutical formulation, such as an oral liquid or an injection.

The present disclosure further provides an anti-tumor drug comprising the pemetrexed disodium liquid composition described above or prepared from the pemetrexed disodium liquid composition described above. For example, the anti-tumor drug can be an oral liquid or an injection.

The present disclosure further provides a method for treating a tumor by providing a therapeutically effective amount of the pemetrexed disodium liquid composition or the anti-tumor drug.

The preferred conditions described above may be combined arbitrarily to give preferred embodiments of the present disclosure without departing from the general knowledge in the art.

The reagents and starting materials used in the present disclosure are commercially available. Beneficial Effects of Present Disclosure:
The pemetrexed disodium liquid composition of the present disclosure has the advantages of simple preparation process, good physical and chemical stability, convenient clinical use, no need of reconstitution, low microbial contamination risk in the preparation process, suitability for industrial production and the like.

In the prior art, the pemetrexed lyophilized powder injection needs to be reconstituted before use and then is subjected to a second dilution for administration, the operation is complicated, the reconstitution process may cause the administration dose difference of patients and the risk of microbial contamination, and the like. These defects can be effectively overcome in the present disclosure.

### BRIEF DESCRIPTION OF THE DRAWINGS

FIG. 1 is a graph of the mean drug concentration-time curves (N = 6) after intravenous injection of formula 16 and the control drug ALIMTA^{®} in beagle dogs, wherein 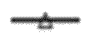 represents the mean drug concentration-time curve of formula 16, and 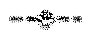 represents the mean drug concentration-time curve after the control drug ALIMTA^{®}.
FIG. 2 shows a trend of PMQS-IM-B impurity in the accelerated experiment of formula 16.
FIG. 3 shows a trend of PMQS-IM-C impurity in the accelerated experiment of formula 16.
FIG. 4 shows a trend of PMQS-IM-F impurity in the accelerated experiment of formula 16.
FIG. 5 shows a trend of PMQS-IM-B impurity in the long-term experiment of formula 16.
FIG. 6 shows a trend of PMQS-IM-C impurity in the long-term experiment of formula 16.
FIG. 7 shows a trend of PMQS-IM-F impurity in the long-term experiment of formula 16.

### DETAILED DESCRIPTION

The present disclosure is further illustrated by the following examples; however, these examples should not be construed as limiting the present disclosure. Experimental procedures without specified conditions in the following examples are conducted in accordance with conventional procedures and conditions, or in accordance with the manufacturer's manual.

The amount of the pemetrexed disodium referred to in the formulas of the examples and comparative examples are based on pemetrexed disodium anhydrate.

### Comparative Example 1

| Component | Formula 1 |
|---|---|
| Pemetrexed disodium mg | 25 |
| Sodium hydroxide | qs pH 8.0~9.0 |
| Water for injection mL | q.s. 1 |

The drug liquid was prepared according to the amount of formula 1 and pre-filtered by a 0.22 µm filter. The resulting solution was placed into a cleaned and sterilized vial and sealed.

### Example 1

| Component | Formula 2 |
|---|---|
| Pemetrexed disodium mg | 25 |
| Tromethamine mg | 2.4 |
| Citric acid | qs pH 8.0~9.0 |
| Water for injection mL | q.s. 1 |

The drug liquid was prepared according to the amount of formula 2 and pre-filtered by a 0.22 µm filter. The resulting solution was placed into a cleaned and sterilized vial, purged with nitrogen and sealed with the headspace residual oxygen < 4%.

### Example 2

The samples obtained by formulas 1-2 were subjected to high temperature/illumination conditions (high temperature: 40 ± 2 °C/75% ± 5% RH, illumination: total illumination 1.2 × 10⁵ lux▪hr, near ultraviolet energy 24w▪hr/m²) to investigate the stability of the system, and the appearance, pH and related substances of the sample solution were detected. The data are summarized as follows:

| Sample | Condition | Appearance | pH | Maximum single impurity% | Total impurity% |
|---|---|---|---|---|---|
| Formula 1 | Day 0 | Colorless and clear solution | 8.72 | 0.05 | 0.2 |
| | Under illumination for 5 days | Yellow and clear solution | 7.02 | 0.55 | 3.07 |
| | Under illumination for 10 days | Yellow and clear solution | 6.97 | 0.84 | 3.65 |
| | 40 °C, 5 days | Yellowish and clear solution | 6.99 | 0.37 | 1.63 |
| | 40 °C, 10 days | Slightly yellowish-green and clear solution | 6.96 | 0.72 | 3.08 |
| Formula 2 | Day 0 | Colorless and clear solution | 8.47 | 0.06 | 0.19 |
| | Under illumination for 5 days | Light yellow and clear solution | 8.43 | 0.08 | 0.37 |
| | Under illumination for 10 days | Yellow and clear solution | 8.45 | 0.11 | 0.48 |
| | 40 °C, 5 days | Colorless and clear solution | 8.44 | 0.04 | 0.16 |
| | 40 °C, 10 days | Colorless and clear solution | 8.45 | 0.03 | 0.17 |

The solution appearance results show that in formula 1, the pemetrexed solution was directly subjected to filling, and the appearance of the solution changed under the conditions of high temperature and illumination; in formula 2, stabilizers of tromethamine and citric acid were added into the formula, and the appearance of the solution changed only under illumination.

The pH results show that the pH of formula 1 was decreased under the conditions of high temperature and illumination, and the maximum decrease was 1.7; the pH of formula 2 was stable under the conditions of high temperature and illumination.

The related substance results show that the total impurity of formula 1 was significantly increased under the conditions of high temperature and illumination; although the total impurity of formula 2 was increased, the increase trend was lower than that of formula 1.

In conclusion, compared with formula 1, the solution of formula 2 had significantly improved physical stability and chemical stability after a stabilizer was added and nitrogen was introduced.

### Example 3

| Component | Formula 3 | Formula 4 | Formula 5 | Formula 6 | Formula 7 |
|---|---|---|---|---|---|
| Pemetrexed disodium mg | 25 | 25 | 25 | 27.57 | 27.57 |
| Tromethamine mg | 2.4 | 2.4 | 2.4 | 2.4 | 2.4 |
| Anhydrous sodium sulfite mg | 1 | NA | NA | 1 | NA |
| Acetyl cysteine mg | NA | 1.63 | NA | NA | NA |
| Cysteine hydrochloride mg | NA | NA | 0.3 | NA | NA |
| Sodium thiosulfate mg | NA | NA | NA | NA | 1 |
| Citric acid | qs pH 8.0~9.0 | | | | |
| Water for injection mL | q.s. 1 | q.s. 1 | q.s. 1 | q.s. 1 | q.s. 1 |

The drug liquid was prepared according to the amount of formulas 3-7 and pre-filtered by a 0.22 µm filter. The resulting solution was placed into a cleaned and sterilized vial, purged with nitrogen and sealed with the headspace residual oxygen < 4%.

### Example 4

The samples obtained by formulas 3-5 were subjected to high temperature/illumination conditions (high temperature: 40 ± 2 °C/75% ± 5% RH, illumination: total illumination 1.2 × 10⁵ lux▪hr, near ultraviolet energy 24w▪hr/m²) to investigate the stability of the system, and the appearance, pH and related substances of the sample solution were detected and compared with those of formula 2. The data are summarized as follows:

| Sample | Antioxidant | Condition | Appearance | pH | Maximum single impurity% | Total impurity% |
|---|---|---|---|---|---|---|
| Formula 2 | NA | Day 0 | Colorless and clear solution | 8.47 | 0.06 | 0.19 |
| | | Under illumination for 5 days | Light yellow and clear solution | 8.43 | 0.08 | 0.37 |
| | | Under illumination for 10 days | Yellow and clear solution | 8.45 | 0.11 | 0.48 |
| | | 40 °C, 5 days | Colorless and clear solution | 8.44 | 0.04 | 0.16 |
| | | 40 °C, 10 days | Colorless and clear solution | 8.45 | 0.03 | 0.17 |
| Formula 3 | Anhydrous sodium sulfite | Day 0 | Colorless and clear solution | 9.05 | 0.04 | 0.21 |
| | | Under illumination for 5 days | Colorless and clear solution | 8.91 | 0.11 | 0.58 |
| | | Under illumination for 10 days | Colorless and clear solution | 8.96 | 0.14 | 0.63 |
| | | 40 °C, 5 days | Colorless and clear solution | 8.85 | 0.13 | 0.6 |
| | | 40 °C, 10 days | Colorless and clear solution | 8.86 | 0.11 | 0.53 |
| Formula 4 | Acetyl cysteine | Day 0 | Colorless and clear solution | 8.07 | 0.04 | 0.16 |
| | | Under illumination for 5 days | Colorless and clear solution | 8.00 | 0.24 | 0.87 |
| | | Under illumination for 10 days | Colorless and clear solution | 7.98 | 0.18 | 0.88 |
| | | 40 °C, 5 days | Colorless and clear solution | 7.94 | 0.07 | 0.36 |
| | | 40 °C, 10 days | Colorless and clear solution | 7.93 | 0.1 | 0.55 |
| Formula 5 | Cysteine hydrochloride | Day 0 | Colorless and clear solution | 8.71 | 0.04 | 0.18 |
| | | Under illumination for 5 days | Colorless and clear solution | 8.64 | 0.07 | 0.29 |
| | | Under illumination for 10 days | Colorless and clear solution | 8.73 | 0.06 | 0.32 |
| | | 40 °C, 5 days | Colorless and clear solution | 8.62 | 0.06 | 0.25 |
| | | 40 °C, 10 days | Colorless and clear solution | 8.66 | 0.05 | 0.27 |

The solution appearance results show that in formula 2, an antioxidant was not added, and the color of the system changed to a certain extent after the system was placed under the illumination condition for a period of time; different types of antioxidants were added into formulas 3-5 and placed for 5 days and 10 days under high temperature and illumination, and the appearance was not changed.

The pH results show that the pH values of the system were relatively stable after different antioxidants were added.

The related substance results show that the total impurity of formula 2 (without adding antioxidant) was substantially kept unchanged under the high temperature condition; in formulas 3-5, the impurity of formula 3 (the antioxidant was anhydrous sodium sulfite) and formula 4 (the antioxidant was acetyl cysteine) increased rapidly; the single impurity of formula 5 (the antioxidant was cysteine hydrochloride) was 0.06% or less, while the total impurity was only 0.32% or less, showing better stability.

**In** conclusion, in the formula with an antioxidant, cysteine hydrochloride was added into the pemetrexed solution as an antioxidant, and the stability of the solution was relatively good; meanwhile, good stability of the formula without adding an antioxidant can be ensured under the high temperature condition, and the single impurity detection amount was low.

### Example 5

| Component | Formula 8 | Formula 9 |
|---|---|---|
| Pemetrexed disodium mg | 25 | 25 |
| Meglumine mg | 2.4 | NA |
| Arginine mg | NA | 2.4 |
| Cysteine hydrochloride mg | 0.3 | 0.3 |
| Citric acid | qs pH 8.0~9.0 | |
| Water for injection mL | q.s. 1 | q.s. 1 |

The drug liquid was prepared according to the amount of formulas 8-9 and pre-filtered by a 0.22 µm filter. The resulting solution was placed into a cleaned and sterilized vial, purged with nitrogen and sealed with the headspace residual oxygen < 4%.

### Example 6

The samples obtained by formulas 8-9 were subjected to high temperature/illumination conditions (high temperature: 40 ± 2 °C/75% ± 5% RH, illumination: total illumination 1.2 × 10⁵ lux▪hr, near ultraviolet energy 24w▪hr/m²) to investigate the stability of the system, and the appearance, pH and related substances of the sample solution were detected and compared with those of formula 5 (the pH adjuster was tromethamine). The data are summarized as follows:

| Sample | Basic pH adjuster | Condition | Appearance | pH | Maximum single impurity% | Total impurity% |
|---|---|---|---|---|---|---|
| Formula 8 | Meglumine | Day 0 | Colorless and clear solution | 8.59 | 0.05 | 0.22 |
| | | Under illumination for 5 days | Colorless and clear solution | 8.54 | 0.06 | 0.29 |
| | | Under illumination for 10 days | Colorless and clear liquid | 8.5 | 0.07 | 0.33 |
| | | 40 °C, 5 days | Colorless and clear solution | 8.53 | 0.06 | 0.23 |
| | | 40 °C, 10 days | Colorless and clear solution | 8.52 | 0.06 | 0.29 |
| Formula 9 | Arginine | Day 0 | Colorless and clear solution | 8.75 | 0.05 | 0.21 |
| | | Under illumination for 5 days | Colorless and clear solution | 8.73 | 0.06 | 0.27 |
| | | Under illumination for 10 days | Colorless and clear liquid | 8.7 | 0.05 | 0.27 |
| | | 40 °C, 5 days | Colorless and clear solution | 8.73 | 0.06 | 0.25 |
| | | 40 °C, 10 days | Colorless and clear solution | 8.73 | 0.08 | 0.3 |
| Formula 5 | Tromethamine | Day 0 | Colorless and clear solution | 8.71 | 0.04 | 0.18 |
| | | Under illumination for 5 days | Colorless and clear liquid | 8.64 | 0.07 | 0.29 |
| | | Under illumination for 10 days | Colorless and clear liquid | 8.73 | 0.06 | 0.32 |
| | | 40 °C, 5 days | Colorless and clear solution | 8.62 | 0.06 | 0.25 |
| | | 40 °C, 10 days | Colorless and clear solution | 8.66 | 0.05 | 0.27 |

It can be seen from the above results that when pH adjusters meglumine, arginine, or tromethamine was added into the formulas, the samples were placed under the conditions of 40 °C high temperature/illumination for 5 days and 10 days, the appearance of the system was not changed, the single impurity was 0.08% or less, and meanwhile the total impurity was only 0.33% or less, indicating that meglumine, arginine, and tromethamine have a good stabilizing effect on the pemetrexed solution and play a role of a stabilizer.

### Example 7

| Component | Formula 10 | Formula 11 | Formula 12 |
|---|---|---|---|
| Pemetrexed disodium mg | 27.57 | 27.57 | 27.57 |
| Tromethamine mg | 2.4 | 2.4 | 2.4 |
| Citric acid | qs pH 8.0~9.0 | qs pH 8.0~9.0 | qs pH 8.0~9.0 |
| Propylene glycol mg | 90 | 180 | 260 |
| Water for injection mL | q.s. 1 | q.s. 1 | q.s. 1 |

The drug liquid was prepared according to the amount of formulas 10-12 and pre-filtered by a 0.22 µm filter. The resulting solution was placed into a cleaned and sterilized vial, purged with nitrogen and sealed with the headspace residual oxygen < 4%.

The samples obtained by formulas 10-12 were subjected to a high temperature condition (high temperature: 40 ± 2 °C/75% ± 5% RH) to investigate the stability of the system, and the related substances of the sample solution were detected and compared with those of formula 5 (the pH adjuster was tromethamine). The data are summarized as follows:

| Formula | Formula difference | Condition | Maximum unknown single impurity | Total impurity ≤ 1.30% | Impurity increase% |
|---|---|---|---|---|---|
| 10 | Propylene glycol 90 mg/mL | Day 0 | 0.07 | 0.26 | 0.13 |
| | | 40 °C, 10 days | 0.07 | 0.39 | |
| 11 | Propylene glycol 180 mg/mL | Day 0 | 0.07 | 0.25 | 0.08 |
| | | 40 °C, 10 days | 0.07 | 0.33 | |
| 12 | Propylene glycol 260 mg/mL | Day 0 | 0.07 | 0.26 | 0.08 |
| | | 40 °C, 10 days | 0.08 | 0.34 | |
| 5 | Cysteine hydrochloride 0.3 mg/mL | Day 0 | 0.04 | 0.18 | 0.09 |
| | | 40 °C, 10 days | 0.05 | 0.27 | |

It can be seen from the above results that when propylene glycol, an organic solvent, was added to a formula to 180 mg/mL or more, the stability was comparable to the related substances increase of formula 5 in which an antioxidant was added. It indicates that the addition of propylene glycol in the formula also has a stabilizing effect on the product.

### Example 8

| Component | Formula 13 | Formula 14 | Formula 15 |
|---|---|---|---|
| Pemetrexed disodium mg | 27.57 | 27.57 | 27.57 |
| Tromethamine mg | 2.4 | 2.4 | 2.4 |
| Citric acid | qs pH 8.0~9.0 | qs pH 8.0~9.0 | qs pH 8.0~9.0 |
| PEG400 mg | 90 | 180 | 280 |
| Water for injection mL | q.s. 1 | q.s. 1 | q.s. 1 |

The drug liquid was prepared according to the amount of formulas 13-15 and pre-filtered by a 0.22 µm filter. The resulting solution was placed into a cleaned and sterilized vial, purged with nitrogen and sealed with the headspace residual oxygen < 4%.

### Example 9

| Component | Formula 16 | Formula 17 | Formula 18 | Formula 19 |
|---|---|---|---|---|
| Pemetrexed disodium mg | 27.57 | 27.57 | 27.57 | 27.57 |
| Tromethamine mg | 2.4 | 2.4 | 2.4 | 2.4 |
| Citric acid monohydrate | qs pH 8.0~9.0 | qs pH 8.0~9.0 | qs pH 8.0~9.0 | qs pH 8.0~9.0 |
| Sodium chloride mg | 5.8 | NA | NA | NA |
| Mannitol mg | NA | 23 | NA | NA |
| Glycerol mg | NA | NA | 15 | NA |
| Propylene glycol mg | NA | NA | NA | 11 |
| Water for injection mL | q.s. 1 | q.s. 1 | q.s. 1 | q.s. 1 |

The drug liquid was prepared according to the amount of formulas 16-19 and pre-filtered by a 0.22 µm filter. The resulting solution was placed into a cleaned and sterilized vial, purged with nitrogen and sealed with the headspace residual oxygen < 4%.

### Example 10

The samples obtained by formulas 16-19 were subjected to a high temperature condition (high temperature: 40 ± 2 °C/75% ± 5% RH) to investigate the stability of the system, and the data of the related substances are summarized as the following table:

| Sample | Osmotic pressure regulator | Condition | Maximum single impurity% | Total impurity% |
|---|---|---|---|---|
| Formula 16 | Sodium chloride | Day 0 | 0.08 | 0.25 |
| | | 40 °C, 10 days | 0.11 | 0.42 |
| Formula 17 | Mannitol | Day 0 | 0.08 | 0.25 |
| | | 40 °C, 10 days | 0.11 | 0.46 |
| Formula 18 | Glycerol | Day 0 | 0.08 | 0.25 |
| | | 40 °C, 10 days | 0.11 | 0.45 |
| Formula 19 | Propylene glycol | Day 0 | 0.08 | 0.22 |
| | | 40 °C, 10 days | 0.12 | 0.54 |

As can be seen from the above table, different types of osmotic pressure regulators have no significant effect on the stability of the product.

In addition, sample solutions were prepared according to the dilution factor in clinical use and tested for osmotic pressure, with the results shown in the following table:

| Dilution factor | Osmotic pressure mOsmol/kg | | | |
|---|---|---|---|---|
| | Formula 16 | Formula 17 | Formula 18 | Formula 19 |
| | Sodium chloride | Mannitol | Glycerol | Propylene glycol |
| Stock solution | 305 | 249 | 289 | 273 |
| 5-fold dilution | 287 | 273 | 280 | 278 |
| 25-fold dilution | 276 | 272 | 274 | 273 |

The results show that the samples prepared in this example can meet the requirement of isotonicity under the clinical intended use.

### Example 11

The drug liquid was prepared according to the formula amount of formula 16 and placed into a colorless neutral borosilicate glass injection vial, and nitrogen was introduced into the solution to reduce the amount of the dissolved oxygen. The oxygen content in the solution was measured and the results are shown in the following table:

| Nitrogen introducing mode | Amount of dissolved oxygen mg/L |
|---|---|
| Without nitrogen introducing | 7.68 |
| Nitrogen introduced into the solution | 4.86 |
| Nitrogen introduced into the solution | 2.90 |

The samples obtained after ampoule sealing were subjected to a high temperature condition (high temperature: 40 ± 2 °C/75% ± 5% RH) to investigate the stability of the system, and the data of the appearance and the related substances are summarized as the following table:

| Difference | Condition | Appearance | Related substance | |
|---|---|---|---|---|
| | | | Maximum single impurity | Total impurity% |
| / | Day 0 | Colorless and clear solution | 0.04 | 0.19 |
| Without nitrogen introducing Amount of dissolved oxygen 7.68 mg/L | 40 °C-5 days | Colorless and clear liquid | 0.09 | 0.38 |
| | 40 °C-10 days | Yellowish and clear liquid | 0.26 | 0.66 |
| Liquid preparation nitrogen introducing Amount of dissolved oxygen 4.86 mg/L | 40 °C-5 days | Colorless and clear liquid | 0.12 | 0.43 |
| | 40 °C-10 days | Yellowish and clear liquid | 0.19 | 0.52 |
| Liquid preparation nitrogen introducing Amount of dissolved oxygen 2.90 mg/L | 40 °C-5 days | Colorless and clear liquid | 0.06 | 0.31 |
| | 40 °C-10 days | Yellowish and clear liquid | 0.16 | 0.46 |

It can be seen from the above results that when the amount of dissolved oxygen in the solution was reduced, the total impurity increase of the pemetrexed disodium solution was smaller, indicating that the amount of dissolved oxygen in the solution has a certain effect on the stability of pemetrexed disodium. Therefore, in order to reduce the effect of the amount of dissolved oxygen in water, highpurity nitrogen can be introduced into the water for injection in the solution preparation process to replace the dissolved oxygen in the water, so that the effect of the dissolved oxygen on the pemetrexed disodium solution was reduced.

### Example 12

| | | |
|---|---|---|
| Component | Formula 20 | |
| Pemetrexed disodium mg | 25 | |
| Tromethamine mg | 3 | |
| Propylene glycol mg | 35 | |
| Cysteine hydrochloride mg | 1 | |
| Citric acid/sodium citrate | qs pH 8.0~9.0 | |
| Water for injection mL | 1 | |
| Process | Without nitrogen introducing | Nitrogen introducing |

The drug liquid was prepared according to the formula amount in the above table and placed into a colorless neutral borosilicate glass injection vial, and whether nitrogen introducing in the headspace had an effect on the stability was investigated.

The prepared samples were subjected to a high temperature condition (high temperature: 40 ± 2 °C/75% ± 5% RH) to investigate the stability of the system, and the appearance of the sample solution and the related substances were detected. The results are shown in the following table:

| Sample | Condition | Appearance | Related substance | |
|---|---|---|---|---|
| | | | Maximum single impurity | Total impurity% |
| Without nitrogen introducing | Day 0 | Colorless and clear solution | 0.07 | 0.29 |
| | 40 °C, 5 days | Colorless and clear solution | 0.13 | 0.66 |
| | 40 °C, 10 days | Yellowish and clear liquid | 0.12 | 0.57 |
| Nitrogen introducing | Day 0 | Colorless and clear solution | 0.07 | 0.29 |
| | 40 °C, 5 days | Colorless and clear solution | 0.07 | 0.27 |
| | 40 °C, 10 days | Colorless and clear liquid | 0.11 | 0.31 |

The solution appearance results show that the appearance of the solution began to change when a sample without nitrogen introducing was placed for 10 days under a high temperature condition, which was changed from colorless and clear liquid into yellowish and clear liquid; after a headspace nitrogen introducing process was added, the appearances of solution systems with different residual oxygen amounts were not significantly changed after the systems were placed for different time under the high temperature condition, which were colorless and clear solutions.

The test results of the related substances show that the total impurity of the headspace nitrogenintroduced sample was 0.31% at 40 °C for 10 days, and the total impurity growth was 0.26% less than that of the sample without headspace nitrogen introducing. Therefore, headspace nitrogen introducing for a product is needed.

Formula 5 was investigated for the headspace nitrogen introducing to determine the limit of the headspace residual oxygen.

| Component | Formula 5 | |
|---|---|---|
| Pemetrexed disodium mg | 25 | |
| Tromethamine mg | 2.4 | |
| Cysteine hydrochloride mg | 0.3 | |
| Citric acid | qs pH 8.0~9.0 | |
| Water for injection mL | q.s. 1 | |
| Headspace residual oxygen% | 3.9 | 1.8 |

The drug liquid was prepared according to the formula amount in the above table and pre-filtered by a 0.22 µm filter. The resulting solution was placed into a cleaned and sterilized vial, and the effect on the stability under different nitrogen introducing processes was investigated.

The prepared samples with different residual oxygen amounts were subjected to a high temperature condition (high temperature: 40 ± 2 °C/75% ± 5% RH) to investigate the stability of the system, and the appearance of the sample solution and the related substances were detected. The results are shown in the following table:

| Headspace residual oxygen (%) | Condition | Appearance | Maximum single impurity (%) | Total impurity% |
|---|---|---|---|---|
| 3.9 | Day 0 | Colorless and clear solution | 0.04 | 0.19 |
| | 40 °C, 5 days | Colorless and clear liquid | 0.05 | 0.23 |
| | 40 °C, 10 days | Colorless and clear liquid | 0.05 | 0.27 |
| 1.8 | Day 0 | Colorless and clear solution | 0.04 | 0.19 |
| | 40 °C, 5 days | Colorless and clear liquid | 0.05 | 0.21 |
| | 40 °C, 10 days | Colorless and clear liquid | 0.05 | 0.24 |

The total impurity increase of the samples with headspace residual oxygen of 3.9% and 1.8% was relatively small, both of which were similar to the results on day 0, and the difference between the two groups was small.

In conclusion, the nitrogen introducing process had a great effect on the stability of the system, and when the headspace residual oxygen was controlled 4% or less, the stability of the system was relatively good.

### Example 13

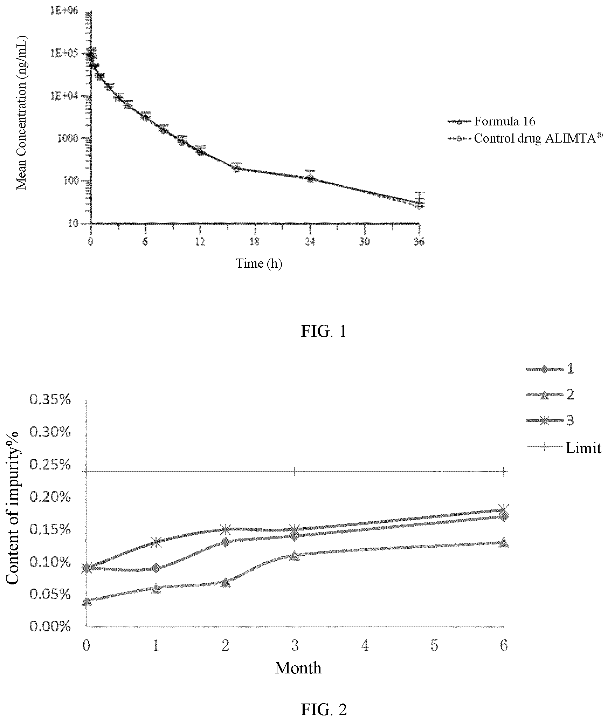

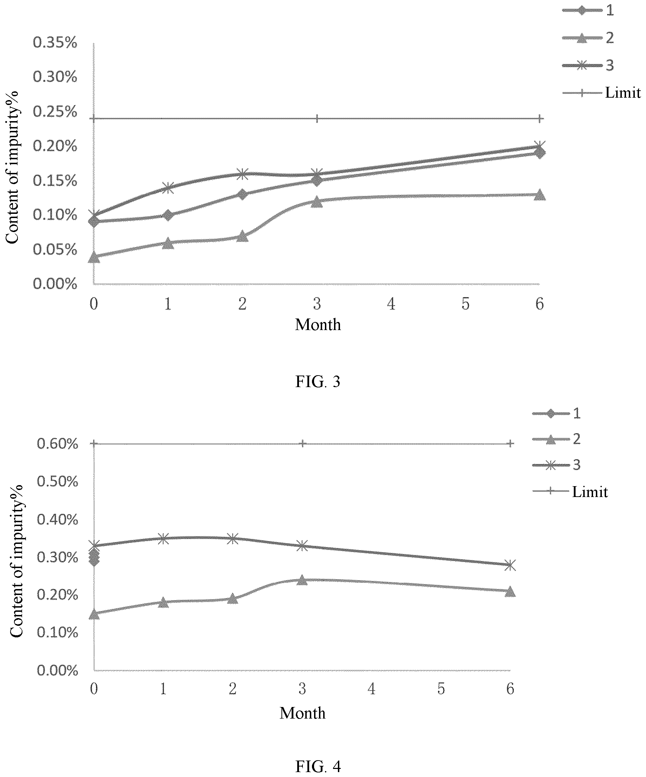

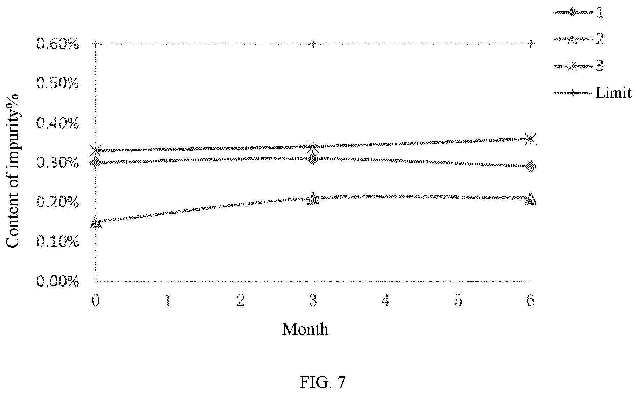

With formula 16 and a corresponding process: before solution preparation, nitrogen was introduced into water for injection to replace oxygen; the headspace residual oxygen after vacuumizing by a lyophilizer and nitrogen introducing was controlled within 4%, three batches of products were produced commercially, and the code numbers were 1, 2, and 3, respectively. The three batches of samples were separately placed under an accelerated condition (25 °C/60% RH) and a long-term condition (5 ± 3 °C). The results of the detection of the related substances are shown in the following Tables A-B and FIGs. 2-7.

**Table A**

| Batch No. | Name of impurity | Limit | Accelerated condition (25 °C/60% RH) | | | | |
|---|---|---|---|---|---|---|---|
| | | | 0 | 1 | 2 | 3 | 6 |
| 1 | PMQS-IM-B | ≤0.24% | 0.09% | 0.09% | 0.13% | 0.14% | 0.17% |
| | PMQS-IM-C | ≤0.24% | 0.09% | 0.10% | 0.13% | 0.15% | 0.19% |
| | PMQS-IM-F | ≤0.60% | 0.30% | 0.27% | 0.30% | 0.29% | 0.28% |
| | Total amount of impurity | ≤1.30% | 0.67% | 0.58% | 0.56% | 0.71% | 0.70% |
| 2 | PMQS-IM-B | ≤0.24% | 0.04% | 0.06% | 0.07% | 0.11% | 0.13% |
| | PMQS-IM-C | ≤0.24% | 0.04% | 0.06% | 0.07% | 0.12% | 0.13% |
| | PMQS-IM-F | ≤0.60% | 0.15% | 0.18% | 0.19% | 0.24% | 0.21% |
| | Total amount of impurity | ≤1.30% | 0.42% | 0.43% | 0.33% | 0.64% | 0.53% |
| 3 | PMQS-IM-B | ≤0.24% | 0.09% | 0.13% | 0.15% | 0.15% | 0.18% |
| | PMQS-IM-C | ≤0.24% | 0.10% | 0.14% | 0.16% | 0.16% | 0.20% |
| | PMQS-IM-F | ≤0.60% | 0.33% | 0.35% | 0.35% | 0.33% | 0.28% |
| | Total amount of impurity | ≤1.30% | 0.71% | 0.74% | 0.66% | 0.88% | 0.72% |

As can be seen from Table A and FIGs. 2-4, the 6-month stability data is far within the limit and meets the criteria under an accelerated condition.

**Table B**

| Batch No. | Name of impurity | Limit | Long-term condition (5 ± 3 °C) | | |
|---|---|---|---|---|---|
| | | | 0 | 3 | 6 |
| 1 | PMQS-IM-B | ≤0.24% | 0.09% | 0.10% | 0.10% |
| | PMQS-IM-C | ≤0.24% | 0.09% | 0.11% | 0.10% |
| | PMQS-IM-F | ≤0.60% | 0.30% | 0.31% | 0.29% |
| | Total amount of impurity | ≤1.30% | 0.67% | 0.69% | 0.55% |
| 2 | PMQS-IM-B | ≤0.24% | 0.04% | 0.06% | 0.07% |
| | PMQS-IM-C | ≤0.24% | 0.04% | 0.07% | 0.07% |
| | PMQS-IM-F | ≤0.60% | 0.15% | 0.21% | 0.21% |
| | Total amount of impurity | ≤1.30% | 0.42% | 0.50% | 0.41% |
| 3 | PMQS-IM-B | ≤0.24% | 0.09% | 0.11% | 0.12% |
| | PMQS-IM-C | ≤0.24% | 0.10% | 0.12% | 0.13% |
| | PMQS-IM-F | ≤0.60% | 0.33% | 0.34% | 0.36% |
| | Total amount of impurity | ≤1.30% | 0.71% | 0.75% | 0.72% |

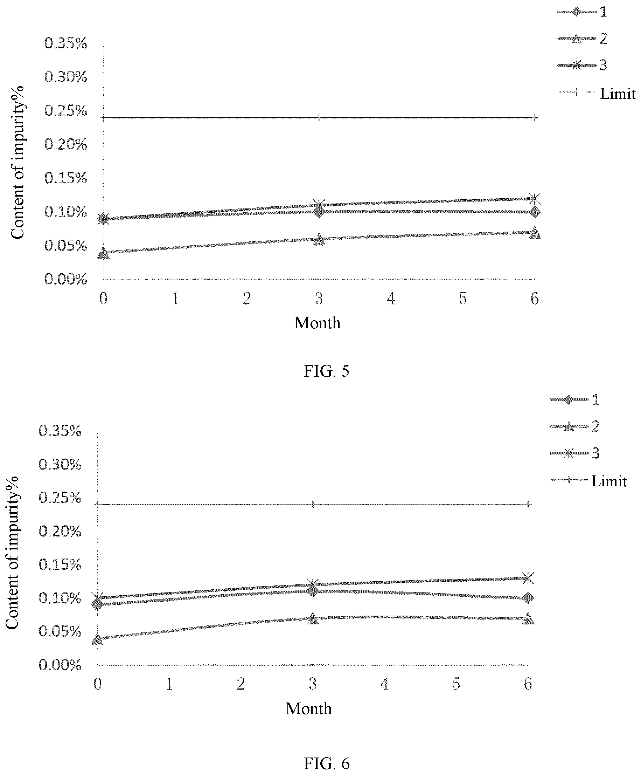

As can be seen from Table B and FIGs. 5-7, under a long-term condition (5 ± 3 °C), the 6-month data shows that the related substances are far within the limit and the stability is good.

According to the above data, the pemetrexed disodium liquid composition of the present disclosure has good stability and stable preparation process, and is suitable for commercial production.

In view of the general principle of making the formula of an injection as simple as possible, formula 16 was selected for investigation of subsequent non-clinical experiments.

### Example 14

In this experiment, 12 beagle dogs in total, half male and half female, were used. Formula 16 and the control drug ALIMTA^{®} were separately intravenously administered to the dogs according to a twocycle crossover experimental design at a dose of 25 mg/kg. Sampling time points: before the administration, immediately after the administration ended (0-1 min), and 5 min, 20 min, 1 h, 2 h, 3 h, 4 h, 6 h, 8 h, 10 h, 12 h, 16 h, 24 h, and 36 h after the administration. The mean pharmacokinetic parameters of pemetrexed in the plasma of the beagle dogs in each group are shown in Table 1, and the mean drug concentration-time curve is shown in FIG. 1.

**Table 1: Mean pharmacokinetic parameters after intravenous injection of formula 16 and the control drug ALIMTA^{®} in beagle dogs (Mean ± SD, N = 12)**

| PK parameters | Formula 16 | Control drug ALIMTA^{®} |
|---|---|---|
| AUClast (h*ug/mL) | 112±17.5 | 116±16.2 |
| AUCINF_obs (h*ug/mL) | 113±17.6 | 117±16.3 |
| Cmax (ug/mL) | 102±23.5 | 116±17.7 |
| Cl_obs (mL/h/kg) | 227±35.4 | 218±31.3 |
| Vss_obs (mL/kg) | 571±73.8 | 528±5.32 |
| Tmax (h) | 0.0278±0.0259 | 0.0167 |
| HL_Lambda_z (h) | 5.79±1.41 | 5.96±1.24 |
| MRTlast (h) | 2.45±0.303 | 2.36±0.281 |
| MRTINF_obs (h) | 2.54±0.329 | 2.45±0.299 |
| C0 (ug/mL) | 109±30.7 | 127±20.8 |

The results described above show that formula 16 achieved similar exposure to that of the control drug and that the key pharmacokinetic parameters were substantially consistent.

### Example 15

The human plasma protein binding rate of formula 16 with different concentrations was assayed by equilibrium dialysis (according to the pharmacokinetic results of beagle dog intravenous injection formula 16 in combination with preclinical experimental data of the reference formulation, this experiment set the experimental groups at low, medium and high concentrations to 10, 100, and 1000 µg/mL, respectively), and compared with the human plasma protein binding rate of the control drug ALIMTA^{®}. The human plasma protein binding rates of formula 16 were 86.50%, 90.19%, and 74.72%, respectively, under the concentrations of 10, 100, and 1000 µg/mL; the human plasma protein binding rates of the control drug (ALIMTA^{®}) at the corresponding concentrations were 86.14%, 89.94%, and 80.96%, respectively. The detail datas of the plasma protein binding rates are shown in Table 2 below.

**Table 2: Human plasma protein binding rates of formula 16 and the control drug with different concentrations**

| Group | | Control drug ALIMTA^{®} | | | | Formula 16 | | | | Positive |
|---|---|---|---|---|---|---|---|---|---|---|
| Addition concentration (µg/mL) | | 10 | 100 | 1000 | PBS | 10 | 100 | 1000 | PBS | Propranolol (5 µM) |
| Administration side (peak area ratio) | N=1 | 12.615 | 1.460 | 15.068 | 1.207 | 10.022 | 0.500 | 5.547 | 0.578 | 3.835 |
| | N=2 | 10.947 | 1.774 | 14.245 | 1.301 | 11.316 | 0.643 | 4.936 | 0.659 | 3.690 |
| | N=3 | 10.968 | 1.659 | 14.501 | 1.281 | 11.013 | 0.621 | 5.192 | 0.702 | 3.798 |
| | Mean | 11.510 | 1.631 | 14.605 | 1.263 | 10.784 | 0.588 | 5.225 | 0.646 | 3.774 |
| | SD | 0.957 | 0.159 | 0.421 | 0.050 | 0.677 | 0.077 | 0.307 | 0.063 | 0.075 |
| Receiving side (peak area ratio) | N=1 | 1.964 | 0.181 | 3.150 | 1.167 | 1.508 | 0.060 | 1.678 | 0.370 | 0.486 |
| | N=2 | 1.456 | 0.164 | 2.306 | 1.097 | 1.398 | 0.059 | 0.964 | 0.427 | 0.480 |
| | N=3 | 1.365 | 0.147 | 2.886 | 1.109 | 1.463 | 0.054 | 3.048* | 0.405 | 0.475 |
| | Mean | 1.595 | 0.164 | 2.781 | 1.124 | 1.456 | 0.058 | 1.321 | 0.401 | 0.480 |
| | SD | 0.323 | 0.017 | 0.432 | 0.037 | 0.055 | 0.003 | / | 0.029 | 0.006 |
| T_{0 h} (peak area ratio) | N=1 | 14.380 | 1.816 | 14.896 | 2.848 | 13.673 | 1.521 | 12.726 | 1.722 | 5.491 |
| | N=2 | 12.173 | 1.900 | 19.708 | 2.488 | 13.854 | 1.265 | 11.464 | 1.555 | 5.043 |
| | N=3 | 12.511 | 1.569 | 16.043 | 2.793 | 13.983 | 1.211 | 13.303 | 1.768 | 5.325 |
| | Mean | 13.021 | 1.762 | 16.882 | 2.710 | 13.837 | 1.332 | 12.498 | 1.682 | 5.286 |
| | SD | 1.189 | 0.172 | 2.513 | 0.194 | 0.156 | 0.166 | 0.941 | 0.112 | 0.226 |
| T_{5 h} (peak area ratio) | N=1 | / | 1.552 | / | 2.490 | / | 1.248 | / | 1.588 | 4.909 |
| | N=2 | / | 1.481 | / | 2.580 | / | 1.851 | / | 1.709 | 4.831 |
| | N=3 | / | 1.380 | / | 2.438 | / | 1.196 | / | 1.759 | 4.947 |
| | Mean | / | 1.471 | / | 2.503 | / | 1.432 | / | 1.685 | 4.896 |
| | SD | / | 0.086 | / | 0.072 | / | 0.364 | / | 0.088 | 0.059 |
| Binding rate (%) | | 86.14 | 89.94 | 80.96 | 10.98 | 86.50 | 90.19 | 74.72 | 38.01 | 87.27 |
| | Mean | 85.68 | | | / | 83.80 | | | / | / |
| | SD | 4.51 | | | / | 8.08 | | | / | / |
| Stability (%) | | / | 83.50 | / | 92.36 | / | 107.46 | / | 100.22 | 92.61 |

The results show that under this experimental condition, the human plasma protein binding rates of pemetrexed disodium in 10 µg/mL, 100 µg/mL, and 1000 µg/mL of formula 16 and the control drug ALIMTA^{®} were all at a medium binding level (50%-90%).

### Example 16

Pemetrexed disodium is a multi-target antimetabolite anti-tumor drug, and is clinically diluted with a 0.9% sodium chloride injection for intravenous infusion. *In vitro* hemolysis experiment was performed on formula 16.

The experiment was carried out by setting a negative control group (0.9% sodium chloride injection, tube 1), a positive control group (sterile water for injection, tube 2), a control drug ALIMTA^{®} group (pemetrexed disodium for injection with the administration concentration of 10 mg/mL, 0.1, 0.2, 0.3, 0.4, and 0.5 mL/tube, respectively, tubes 3-7), and a formula 16 group (with the administration concentration of 10 mg/mL, 0.1, 0.2, 0.3, 0.4, and 0.5 mL/tube, respectively, tubes 8-12), 3 parallel tubes for each group. 2% red blood cell suspension, 0.9% sodium chloride injection, sterile water for injection, the control drug ALIMTA^{®} and formula 16 groups were mixed well according to a set proportion, placed in an incubator, left to stand within the temperature range of 37 ± 0.5 °C, and separately observed once at 0, 15, 30, 45, 60, 120, and 180 minutes (±10%). The specific results are detailed in Table 3.

**Table 3: Results of in vitro hemolysis experiments on different formulations**

| Test tube No. | 2% red blood cell suspension (mL) | 0.9% sodium chloride injection (mL) | Sterile water for injection (mL) | ALIMTA^{®} (mL) | Formula 16 (mL) | 0min | 15min | 30min | 45min | 60min | 120min | 180min |
|---|---|---|---|---|---|---|---|---|---|---|---|---|
| 1-1 | 2.5 | 2.5 | 0 | 0 | 0 | - | - | - | - | - | - | - |
| 1-2 | | | | | | - | - | - | - | - | - | - |
| 1-3 | | | | | | - | - | - | - | - | - | - |
| 2-1 | 2.5 | 0 | 2.5 | 0 | 0 | - | + | ++ | ++ | ++ | ++ | ++ |
| 2-2 | | | | | | - | + | ++ | ++ | ++ | ++ | ++ |
| 2-3 | | | | | | - | + | ++ | ++ | ++ | ++ | ++ |
| 3-1 | 2.5 | 2.4 | 0 | 0.1 | 0 | - | - | - | - | - | - | - |
| 3-2 | | | | | | - | - | - | - | - | - | * |
| 3-3 | | | | | | - | - | - | - | - | - | - |
| 4-1 | 2.5 | 2.3 | 0 | 0.2 | 0 | - | - | - | - | - | - | - |
| 4-2 | | | | | | - | - | - | - | - | - | * |
| 4-3 | | | | | | - | - | - | - | - | - | * |
| 5-1 | 2.5 | 2.2 | 0 | 0.3 | 0 | - | - | - | - | - | - | * |
| 5-2 | | | | | | - | - | - | - | - | - | * |
| 5-3 | | | | | | - | - | - | - | - | - | * |
| 6-1 | 2.5 | 2.1 | 0 | 0.4 | 0 | - | - | - | - | - | - | * |
| 6-2 | | | | | | - | - | - | - | - | - | * |
| 6-3 | | | | | | - | - | - | - | - | - | * |
| 7-1 | 2.5 | 2.0 | 0 | 0.5 | 0 | - | - | - | - | - | - | * |
| 7-2 | | | | | | - | - | - | - | - | - | * |
| 7-3 | | | | | | - | - | - | - | - | - | * |
| 8-1 | 2.5 | 2.4 | 0 | 0 | 0.1 | - | - | - | - | - | - | - |
| 8-2 | | | | | | - | - | - | - | - | - | - |
| 8-3 | | | | | | - | - | - | - | - | - | - |
| 9-1 | 2.5 | 2.3 | 0 | 0 | 0.2 | - | - | - | - | - | - | - |
| 9-2 | | | | | | - | - | - | - | - | - | - |
| 9-3 | | | | | | - | - | - | - | - | - | - |
| 10-1 | 2.5 | 2.2 | 0 | 0 | 0.3 | - | - | - | - | - | - | - |
| 10-2 | | | | | | - | - | - | - | - | - | - |
| 10-3 | | | | | | - | - | - | - | - | - | - |
| 11-1 | 2.5 | 2.1 | 0 | 0 | 0.4 | - | - | - | - | - | - | - |
| 11-2 | | | | | | - | - | - | - | - | - | - |
| 11-3 | | | | | | - | - | - | - | - | - | - |
| 12-1 | 2.5 | 2.0 | 0 | 0 | 0.5 | - | - | - | - | - | - | - |
| 12-2 | | | | | | - | - | - | - | - | - | - |
| 12-3 | | | | | | - | - | - | - | - | - | - |

| | | | | | | | | | | | | |
|---|---|---|---|---|---|---|---|---|---|---|---|---|
| Note: 1) "-" represents "no hemolysis or coagulation", "+" represents "partial hemolysis", "++" represents "complete hemolysis", and "*" represents "coagulation". 2) tube 1 is the negative control group, tube 2 is the positive control group, tubes 3-7 are ALIMTA^{®} groups, and tubes 8-12 are formula 16 groups. The same applies below. | | | | | | | | | | | | |

The results show that: after standing at 37 ± 0.5 °C for 180 min, the red blood cells in the negative control group tube were all sedimented, the supernatant was colorless and clear, the sedimented red blood cells were redispersed after proper shaking, and hemolysis and coagulation were not observed; the solution in the positive control group tube was clear and red, no red blood cell residue was left at the bottom of the tube, and complete hemolysis was observed. The above results suggest that this experimental system is normal and reliable.

The red blood cells in the ALIMTA^{®} group tube were all sedimented, the supernatant was colorless and clear, and hemolysis was not observed, but coagulated red blood cells can still be observed in the sedimented red blood cells and did not disperse after shaking, and the coagulated red blood cells were not broken up by microscopic examination, which was judged not hemolysis but coagulation.

The red blood cells in the formula 16 group tube were all sedimented, the supernatant was colorless and clear, and the sedimented red blood cells were redispersed after proper shaking. No abnormality was observed by microscopic examination, and hemolysis and coagulation were not observed.

In conclusion, the formula 16 group with the concentration of 10 mg/mL had no hemolysis and coagulation effects on rabbit red blood cells, and the *in vitro* hemolysis experiment result was negative.

### Example 17

The product need to be diluted with 0.9% sodium chloride injection for intravenous infusion in clinical use. Active systemic anaphylaxis experiment in guinea pigs was performed on formula 16, and the guinea pigs were observed for anaphylaxis after administration of the test sample, thereby providing reference for evaluating clinical use safety.

The experiment was carried out by setting 6 groups, which were a negative control group, a positive control group, low and high dose ALIMTA^{®} groups, and low and high dose formula 16 groups, respectively, 6 guinea pigs each group, half male and half female. In the sensitization stage, the negative control group was intravenously injected with 0.9% sodium chloride injection according to a volume of 4 mL/kg, the positive control group was intravenously injected with 8 mg/mL egg white albumin solution according to a volume of 0.5 mL/guinea pig, and the low and high dose ALIMTA^{®} and formula 16 groups were intravenously injected with 10 mg/mL of ALIMTA^{®} or formula 16 according to volumes of 2 mL/kg and 4 mL/kg, respectively. The administration doses were 20 m/kg and 40 mg/kg and were about 1.5 and 3 times of the highest clinical intended dose. The day of the first administration was defined as day 1 of the experiment. Sensitization was performed 1 time every other day. 3 consecutive sensitizations were performed (i.e., sensitization on days 1, 3, and 5 of the experiment). On day 14 and day 21 after the last sensitization (i.e., on day 19 and day 26 of the experiment), the guinea pigs were challenged by intravenous injection at 2 times the dose of the sensitization dose, and the systemic reaction and death after the challenge were observed.

**Table 4 Observations of systemic anaphylaxis experiment after first challenge**

| Group | Number of animal (animal) | Number of symptoms of anaphylaxis and occurrence proportion* | | | | | Anaphylaxis Incidence (%) | Anaphylaxis Degree of occurrence |
|---|---|---|---|---|---|---|---|---|
| | | 0 | 1~4 | 5~10 | 11~19 | 20 | | |
| Negative control group | 6 | 6/6 | 0 | 0 | 0 | 0 | 0 | Negative |
| Positive control group | 6 | 0 | 0 | 0 | 0 | 6/6 | 100 | Extremely strong positive |
| ALIMTA^{®} low dose group | 6 | 6/6 | 0 | 0 | 0 | 0 | 0 | Negative |
| ALIMTA^{®} high dose group | 6 | 6/6 | 0 | 0 | 0 | 0 | 0 | Negative |
| Formula 16 low dose group | 6 | 6/6 | 0 | 0 | 0 | 0 | 0 | Negative |
| Formula 16 high dose group | 6 | 6/6 | 0 | 0 | 0 | 0 | 0 | Negative |

| | | | | | | | | |
|---|---|---|---|---|---|---|---|---|
| Note: *the number of anaphylaxis animals was counted as the highest number of stages of anaphylaxis occurring in the animals. The same applies in Table 5. | | | | | | | | |

**Table 5 Observations of systemic anaphylaxis experiment after last challenge**

| Group | Number of animal (animal) | Number of symptoms of anaphylaxis and occurrence proportion | | | | | Anaphylaxis Incidence (%) | Anaphylaxis Degree of occurrence |
|---|---|---|---|---|---|---|---|---|
| | | 0 | 1~4 | 5~10 | 11~19 | 20 | | |
| Negative control group | 6 | 6/6 | 0 | 0 | 0 | 0 | 0 | Negative |
| ALIMTA^{®} low dose group | 6 | 6/6 | 0 | 0 | 0 | 0 | 0 | Negative |
| ALIMTA^{®} high dose group | 6 | 6/6 | 0 | 0 | 0 | 0 | 0 | Negative |
| Formula 16 low dose group | 6 | 6/6 | 0 | 0 | 0 | 0 | 0 | Negative |
| Formula 16 high dose group | 6 | 6/6 | 0 | 0 | 0 | 0 | 0 | Negative |

The results show that during sensitization, the guinea pigs in each group had a good general condition, normal self-activity, clean skin and hair, no abnormal secretion, normal weight increase, and no other abnormal symptoms.

Within 30 minutes after the first challenge, all guinea pigs of the negative control group, the low and high dose ALIMTA^{®} groups and the low and high dose formula 16 groups had no anaphylaxis; the guinea pigs of the positive control group showed allergic symptoms and all died within 7 minutes after administration, and the anaphylaxis was extremely positive.

Within 30 minutes after the last challenge, all guinea pigs of the negative control group, the low and high dose ALIMTA^{®} groups and the low and high dose formula 16 groups had no anaphylaxis.

In summary, under this experimental conditions, the Britain guinea pigs were intravenously injected with 10 mg/mL of formula 16 at volumes of 2 and 4 mL/kg, i.e., at administration doses of 20 and 40 mg/kg, respectively, and the results of the active systemic anaphylaxis experiment were negative.

### Example 18

A rabbit single intravenous injection irritation experiment was carried out on formula 16. The irritant reaction and the reversible degree of the test sample on the ear vein vessels and the surrounding tissues of the rabbit were observed, thereby providing reference for evaluating clinical use safety.

The experiment was carried out by setting 2 groups, which were ALIMTA^{®} group and formula 16 group, respectively, 8 rabbits each group, half male and half female. Each group of rabbits was intravenously injected with 10 mg/mL ALIMTA^{®} or formula 16 via right ear vein at a volume of 4 mL/kg. The administration dose was 40 mg/kg and was about 3 times the highest clinical intended dose. Meanwhile, self same body control was used, and intravenously injected with 0.9% sodium chloride injection via left ear vein at the same volume. A single administration was performed, and observation was performed for 21 consecutive days after administration. The day of the administration was defined as day 1 of the experiment.

During the experiment, the general condition of the rabbits and the condition of the injection site were observed every day. 3 days and 21 days after administration (i.e., day 4 and day 22 of the experiment), 4 rabbits (half male and half female) were taken from each group, euthanized, and dissected. The local irritation responses of the injection was visually observed, and the taken tissues were subjected to pathological examination.

In the observation period after administration, the bilateral injection sites of each group of rabbits had no responses such as redness and swelling, hyperemia, necrosis, and the like, and the rabbits had a good general condition, normal self-activity, and no other abnormal symptoms.

No macroscopic abnormal changes were observed at the bilateral injection sites of each group of rabbits 3 days and 21 days after administration.

3 days after administration, 1 injection site (1/8 ratio) on the control side exhibited slight vascular inflammation associated with mechanical irritation of injection puncture. **In** addition, no abnormality was observed in the remaining injection sites in each group 3 days and 21 days after administration. The results described above show that, under this experimental conditions, the Japanese big-ear white rabbit was intravenously injected with 10 mg/mL of formula 16 via right ear vein at a volume of 4 mL/kg, i.e., at an administration dose of 40 mg/kg, and no irritation was observed to the blood vessel and surrounding tissues at the injection site.

In conclusion, the pemetrexed disodium liquid composition provided by the present disclosure can reach exposure similar to that of a commercially available control, has less in-vivo irritation, has a lower adverse effect, is safer and more convenient, can improve medication compliance of patients and the convenience of clinical administration, and has good market prospects.

Although some embodiments of the present disclosure have been described in detail, those skilled in the art will appreciate that various modifications and changes can be made to the specific embodiments shown without materially departing from the teachings and advantages of the heart of the present disclosure. Such modifications and changes are included within the spirit and scope of the present disclosure to which the appended claims belong.

## Claims

1. A pemetrexed disodium liquid composition comprising an active pharmaceutical ingredient and a stabilizer, wherein the active pharmaceutical ingredient is one or more of pemetrexed disodium, and a pharmaceutically acceptable complex, salt, solvate, and hydrate of pemetrexed disodium; the stabilizer is one or more of an organic solvent, a pH adjuster, and an antioxidant.

2. The pemetrexed disodium liquid composition according to claim 1, wherein the content of the active pharmaceutical ingredient is 1 mg/mL to 50 mg/mL, and the content refers to the ratio of the mass of the active pharmaceutical ingredient to the total volume of the pemetrexed disodium liquid composition.

3. The pemetrexed disodium liquid composition according to claim 1 or 2, wherein
the content of the active pharmaceutical ingredient is 15 mg/mL to 30 mg/mL, and the concentration refers to the ratio of the mass of the active pharmaceutical ingredient to the total volume of the pemetrexed disodium liquid composition;
and/or,
the organic solvent is one or more of ethanol, propylene glycol, and polyethylene glycol;
and/or,
the pH adjuster is an acidic pH adjuster and/or a basic pH adjuster;
and/or,
the antioxidant is one or more of anhydrous sodium sulfite, cysteine hydrochloride, acetyl cysteine, and methionine.

4. The pemetrexed disodium liquid composition according to claim 3, wherein
the acidic pH adjuster is hydrochloric acid and/or citric acid;
and/or,
the basic pH adjuster is one or more of sodium hydroxide, lysine, arginine, meglumine, and tromethamine;
and/or,
the polyethylene glycol is polyethylene glycol 300 and/or polyethylene glycol 400.

5. The pemetrexed disodium liquid composition according to claim 4, wherein
the lysine is L-lysine and/or D-lysine;
and/or,
the arginine is L-arginine and/or D-arginine.

6. The pemetrexed disodium liquid composition according to claim 3, wherein
in the pemetrexed disodium liquid composition, the content of the basic pH adjuster is 0.5 mg/mL to 50 mg/mL, and the content refers to the ratio of the mass of the basic pH adjuster to the total volume of the pemetrexed disodium liquid composition;
and/or,
the pH of the pemetrexed disodium liquid composition is 7.5-9.5;
and/or,
the content of the antioxidant is 0-15 mg/mL, and the content refers to the ratio of the mass of the antioxidant to the total volume of the pemetrexed disodium liquid composition.

7. The pemetrexed disodium liquid composition according to claim 6, wherein
in the pemetrexed disodium liquid composition, the content of the basic pH adjustment is 1 mg/mL to 10 mg/mL, and the content refers to the ratio of the mass of the basic pH adjuster to the total volume of the pemetrexed disodium liquid composition;
and/or,
the pH of the pemetrexed disodium liquid composition is 8.0-9.0;
and/or,
the content of the antioxidant is 0.1 mg/mL to 10 mg/mL, and the content refers to the ratio of the mass of the antioxidant to the total volume of the pemetrexed disodium liquid composition.

8. The pemetrexed disodium liquid composition according to any one of claims 1-7, wherein the pemetrexed disodium liquid composition further comprises an osmotic pressure regulator; preferably, the osmotic pressure regulator is one or more of sodium chloride, mannitol, glycerol, and propylene glycol;
and/or,
the content of the osmotic pressure regulator is 1 mg/mL to 300 mg/mL, and the content refers to the ratio of the mass of the osmotic pressure regulator to the total volume of the pemetrexed disodium liquid composition.

9. A preparation method for the pemetrexed disodium liquid composition according to any one of claims 1-8, wherein the preparation method comprises a nitrogen introducing process;
preferably, the nitrogen introducing process is liquid preparation nitrogen introducing and/or nitrogen filling;
preferably, the nitrogen introducing process is that the content of the dissolved oxygen introduced to the pemetrexed disodium liquid composition via nitrogen introduction is less than 5 mg/L and/or the headspace residual oxygen amount is less than 6%; further preferably, the nitrogen introducing process is that the content of the dissolved oxygen introduced to the pemetrexed disodium liquid composition via nitrogen introduction is less than 3 mg/L and/or the headspace residual oxygen amount is less than 4%.

10. Use of the pemetrexed disodium liquid composition according to any one of claims 1-8 for manufacturing an anti-tumor drug, wherein preferably, the anti-tumor drug is an oral liquid or an injection.
